# EUROPEAN PATENT APPLICATION

(11) **EP 4 079 347 A1**
(43) Date of publication of application: **26.10.2022**
(21) Application number: 21169885.7
(22) Date of filing: 22.04.2021
(51) Int. Cl.: A61M 5/142

(54) **MEDICAL DEVICE SYSTEM AND DISPOSABLE CONTAINER**

(71) Applicant: Roche Diabetes Care GmbH, 68305 Mannheim (DE); F. Hoffmann-La Roche AG, 4070 Basel (CH)
(72) Inventor: VON CAMPENHAUSEN, Harald, 68305 Mannheim (DE)
(74) Representative: Pfaffmann, Lucas

(57) **Abstract**

The present invention relates to a medical device system comprising a body-wearable medical device, and a disposable container comprising a reservoir volume configured for storing a foodstuff or a drug and a tamper evident closure which seals an opening in the disposable container, wherein the opening provides access to the reservoir volume. To reduce the burden of the patient to compose the packages to be carried along by a patient when leaving home, the disposable container comprises a body-wearable medical device fastener configured for detachable fastening the disposable container to the body-wearable medical device and/or the body-wearable medical device comprises a disposable container fastener configured for detachable fastening the body-wearable medical device to the disposable container.

## Description

The present invention relates to a medical device system comprising a body-wearable medical device, and a disposable container comprising a reservoir volume configured for storing a foodstuff or a drug and a tamper evident closure which seals an opening in the disposable container, wherein the opening provides access to the reservoir volume.

Body-wearable medical devices are carried by patients in order to maintain or to improve their health condition in view of a serious illness, which requires the patient to monitor certain body parameter and/or to administer a drug over a long period of time with the help of the body-wearable medical device. For instance, a patient suffering from diabetes has to regularly monitor the blood glucose level and to administer a respective dose of insulin and/or carbohydrates to keep the blood glucose level within a predetermined range.

Sometimes wearing a body-wearable medical device is not enough for a patient, as there might be exceptional situations which cannot be handled by the body-wearable medical device, but which requires the patient to administer an additional amount of drug or to digest an amount of a foodstuff. Accordingly, in addition to the body-wearable medical device the patient has to carry a so-called emergency pack containing an additional amount of drug or an amount of a foodstuff, which have to be administered by the patient in case of emergency. Not having the additional amount of drug or foodstuff at hand may result in a life-threating situation. For instance, a diabetic has to carry a certain amount of carbohydrates, usually sugar, which can be digest in case of an exceptional low blood glucose level, a so-called hypoglycemic state, in order to raise the blood glucose level on a short-notice. Patients that use medical devices generally need to carry a variety of devices and accessories in order to sustain their disease management. For example, a diabetic patient on insulin therapy may carry with him an insulin pump, a replacement infusion set, extra batteries, a glucose meter, glucose meter test strips, a lancet device, an insulin vial, and a carbohydrate foodstuff. This situation is associated with the challenge of having to check and remember which items the patient needs to pack and carry along to ensure that he can manage the disease. If the diabetic patient, e.g., forgets to pack extra insulin there is a risk that the pump's insulin reservoir runs empty and the patient cannot refill the reservoir thus runs the risk to interrupt the insulin therapy which may lead to hyperglycemia. If in another scenario, the patient forgets to carry a carbohydrate containing foodstuff such as a sugar pack or solution, in the event of a hypoglycemic situation the patient is not able to treat that condition by consuming carbohydrates so as to raise the blood level to a normal level. This, in turn, may lead to unconsciousness or even death. Thus, there is a need to increase the safety of the disease management and to alleviate the burden on the patients to remember carrying extra articles important for the disease management.

Accordingly, it is an object of the present invention to reduce the burden of the patient to compose the packages to be carried along by a patient when leaving home.

At least one of the above objects is solved by a medical device system as outlined above, wherein the disposable container comprises a body-wearable medical device fastener configured for detachable fastening the disposable container to the body-wearable medical device and/or the body-wearable medical device comprises a disposable container fastener configured for detachable fastening the body-wearable medical device to the disposable container.

The disposable container contains an amount of a drug or a foodstuff, which can be administered by the patient, if needed. Accordingly, the disposable container comprises a reservoir volume configured for storing a foodstuff or a drug, and having a tamper evident closure. The tamper evident closure seals an opening, which, when opened, provides access to the reservoir volume such that the drug or foodstuff can be withdrawn from the reservoir volume. The tamper evident closure is used to indicate whether or not the opening has been tampered with, i.e. if the seal provided by the tamper evident closure has been broken. Accordingly, once opened the tamper evident closure prevents to reseal the container and preferably also prevents to reuse of the container. It may allow the user to readily and safely determine that the disposable container has been opened and thus helps to prevent use of a potentially contaminated foodstuff or drug. After withdrawal of the drug or the foodstuff from the reservoir volume of the disposable container, the disposable container is disposed. The opening for withdrawing foodstuff or drug from the reservoir volume can be formed by one or more than one opening, such as by at least 2, at least 3, at least 4 or at least 5 openings.

The disposable container comprise a body-wearable medical device fastener configured for detachable fastening the disposable container to the body-wearable medical device, and/or the body-wearable medical device comprises a container fastener configured for detachable fastening the body-wearable medical device to the container.

In a state, in which the body-wearable medical device and the disposable container are mounted to each other, they form a unit, which can be carried by the patient. Thus, by wearing the body-wearable medical device, at the same time, the patient carries the disposable container containing foodstuff or drug to be administered in case of emergency. By being mounted to the body-wearable medical device the disposable container is less likely prone to be forgotten by the patient when packing his disease management devices and accessories because the disposable container is already mounted on the body-wearable medical device when the patient is leaving home, the office, or any other place, where the patient needs to have access to an additional amount of a drug or a foodstuff, which can be administered in case of an emergency.

The mounting of the body-wearable medical device and the disposable container is reversible, such that the disposable container can be mounted to and demounted from the body-wearable medical device. Reversible mounting is convenient for a patient so he can withdraw or administering the drug or foodstuff contained in the reservoir volume of the disposable container without having to remove the body-wearable medical device from the body. Once emptied the disposable container can be disposed and replaced by a new disposable container, which again can be mounted on the body-wearable medical device.

In the meaning of the present invention, the term disposable container refers to a container that is intended for being used for one-time only. I.e. after opening the tamper-evident closure and withdrawing content out of the reservoir, the container is disposed, and should not be re-filled or re-used. Thereby, an abuse of the disposable container is less likely. In particular, once opened the reservoir volume can be contaminated, which may adversely affect the integrity, therapeutic effectivity and hygiene of the drug or foodstuff contents. Thus, the disposable nature of the container and the tamper evident closure sealing the opening of the reservoir volume increase patient's safety.

In general, the foodstuff or drug contained in the reservoir volume of the disposable container can be liquid, pasty, powdery, granular, or a mixture thereof. Accordingly, in an embodiment, the reservoir volume of the disposable container is configured to hold a liquid, a gel, or a pasty or powdery or granular substance, or a mixture thereof. In an embodiment, the reservoir volume of the disposable container contains a foodstuff comprising a carbohydrate which can be in a liquid, a gel, or a pasty or powdery or granular substance form, or a mixture thereof. In an embodiment the foodstuff comprises a carbohydrate the consumption of which increases the blood glucose level. Preferably, the carbohydrate comprises sugar, such as fructose, glucose, or dextrose. In another embodiment the reservoir volume contains a drug which can be in liquid, a gel, or a pasty or powdery or granular substance form, or a mixture thereof. The drug can be any drug that can be stored under temperature conditions in the disposable container of e.g. in a range between 10 degrees to 30 degrees, preferably at room temperature, without significantly adversely affecting the therapeutic efficacy of the stored drug for an interval of time of up to 1 hour, up to 2 hours, up to 5 hours, up to 7 hours, up to 10 hours, up to 1 day, up to 2 days, up to 3 days, up to 5 days, up to 7 days.

In an embodiment the drug is a drug for the treatment of a disease selected from cancer, a chronic disease such as diabetes or asthma, a cardiovascular disease, a metabolic disease, an autoimmune disease or an infectious disease. Preferably the drug is used for treating diabetes, preferably the drug is an insulin. In an embodiment the insulin includes a rapid-acting, a short-acting, an intermediate-acting, a long-acting or a pre-mixed insulin, In an embodiment the insulin is selected from Lispro (Humalog), Aspart (Novolog), Glulisine (Apidra), Regular (R) or Novolin, Velosulin, NPH, Insulin glargine (Basaglar, Lantus, Toujeo), Insulin detemir (Levemir), Insulin degludec (Tresiba), Humulin, and Novolog.

According to an embodiment of the medical device system, the body-wearable medical device fastener is or comprises a mechanical, non-adhesive medical device fastener and/or the disposable container fastener is or comprises a mechanical, non-adhesive disposable container fastener. A mechanical, non-adhesive medical device fastener or a mechanical, non-adhesive disposable container fastener can be removed free of residues. In particular, upon removal of a mechanical, non-adhesive fastener there is no sticky residue left on the disposable container or the body-wearable medical device, respectively, which are otherwise may cause discomfort to a user. In an embodiment, the term non-adhesive fastener is meant to specify that the fastener is free of a substance (such as a glue) for sticking the disposable container and the body-wearable medical device together.

According to an embodiment of the medical device system, the body-wearable medical device fastener is or comprises a medical device bracket configured for at least partially embracing the body-wearable medical device and/or the disposable container fastener is or comprises a disposable container bracket configured for at least partially embracing the container. In the meaning of the present invention a medical device bracket configured for at least partially embracing the body-wearable medical device or disposable container bracket for at least partially embracing the disposable container comprises a first portion and second portion, which are connected to each by a bridging portion such that during use, i.e. when embracing the body-wearable medical device or disposable container, respectively, the first portion and second portion of the respective bracket are arranged on opposing sides of the body-wearable medical device or disposable container, respectively. The first portion, second portion, and bridging portion can be configured to provide a clamping force for detachable fastening the body-wearable medical device to the disposable container, or vice versa. The first portion and second portion each can be a part of respective pairs of correspondingly configured fastener pairs as for instance described below.

According to an embodiment of the medical device system, the disposable container comprises a circumferential edge, which, in a state of the disposable container fastened to the body-wearable medical device, circumferentially contacts the body-wearable medical device. The circumferential edge of the container contacting the body-wearable medical device stabilize the connection between the body-wearable medical device and the container. Furthermore, the contacting circumferential edge prevent foreign matter entering between the body-medical device and the container. In a further modified embodiment, the circumferential edge is configured for clamping the disposable container to the body-wearable medical device thereby being configured as the body-wearable medical device fastener or at least part of the body-wearable medical device fastener.

According to an embodiment of the medical device system, the disposable container fastener and the body-wearable medical device fastener interoperate to detachably fasten the disposable container to the body-wearable medical device. The disposable container fastener and the body-wearable medical device fastener form a pair of engaging mechanical elements. In a state, the disposable container and the body-wearable medical device are detached from each other, the disposable container fastener remains at the body-wearable medical device, while the body-wearable medical device fastener remains at the disposable container. According to a further modified embodiment, the interoperating disposable container fastener and body-wearable medical device fastener are configured to allow fastening the disposable container to the body-wearable medical device in a single pre-determined position defined by the respective arrangement of the interoperating disposable container fastener and body-wearable medical device fastener.

According to an embodiment of the medical device system, the disposable container fastener and the body-wearable medical device fastener form a pair of hook-and-loop fastener or a pair of hook-and-eye fastener or a latch fastener or clip fastener.

According to an embodiment of the medical device system, the reservoir volume is up to 20 cm³, alternatively the reservoir volume is up to 15 cm³, further alternatively the reservoir volume is equal to 13 cm³. In any case the reservoir volume is larger 0 cm³, so that the reservoir volume can contain a foodstuff or a drug.

In order to ease withdrawal of a foodstuff or a drug contained in the reservoir volume of the disposable container via the opening, according to an embodiment of the medical device system, the disposable container comprises a rip-open venting closure sealing a venting opening of the reservoir. This additional venting opening allows venting the reservoir of the disposable once the rip-open venting closure is opened such that withdrawal of foodstuff or drug via the opening of the disposable container is not hindered by negative pressure or a reverse flow of air trying to enter the reservoir volume through the opening for withdrawing foodstuff or drug. Such venting opening is particularly useful if the foodstuff or liquid contained in the reservoir volume is in liquid or gel form. A rip-open closure can be opened without using any tools. The rip-open venting closure that seals the venting open and the tamper-evident closure sealing the opening for withdrawing foodstuff or drug from the reservoir volume can be a combined in one closure. I.e. the venting closure and the tamper-evident closure can be removed together so that the venting opening and the opening for withdrawing foodstuff or drug from the reservoir volume are opened together.

In an embodiment of the medical device system the disposable container, except of the body-wearable medical device fastener, is an envelope or pouch, preferably the envelope or pouch is pre-filled with a foodstuff or a drug. In the meaning of the present invention, an envelope or pouch is a flexible container. The wall thickness of the envelope or pouch can be equal to or smaller than 1 mm. The body-wearable medical device fastener can be but do not need to be flexible like the remainder of the disposable container. In an embodiment, the disposable container is entirely or at least partially made of a plastic. For instance, the plastic is or comprises polycarbonate, polyethylene, polypropylene, or a mixture thereof.

The present invention further relates to a disposable container, wherein an object of the present invention is solved by a disposable container comprising a reservoir volume configured for storing a foodstuff or a drug, and a tamper evident closure which seals an opening in the disposable container, the opening provides access to the reservoir volume, wherein the disposable container comprises a body-wearable medical device fastener configured for detachable fastening the disposable container to a body-wearable medical device. The disposable container can be configured as described above in the context of the medical device system. By way of example only, the body-wearable medical device can be configured as described in the context of the medical device system above, or as described further below.

According to an embodiment of the disposable container, the body-wearable medical device fastener is or comprises a mechanical, non-adhesive medical device fastener.

According to an embodiment of the disposable container, the body-wearable medical device fastener is or comprises a medical device bracket configured for at least partially embracing the body-wearable medical device.

According to an embodiment of the disposable container, the disposable container comprises a circumferential edge, which, in a state of the disposable container fastened to the body-wearable medical device, circumferentially contacts the body-wearable medical device.

According to an embodiment of the disposable container, the body-wearable medical device fastener is configured to interoperate with a disposable container fastener of the body-wearable medical device to detachably fasten the disposable container to the body-wearable medical device.

According to an embodiment of the disposable container, the body-wearable medical device fastener is a portion of a pair of hook-and-loop fastener or a portion of a pair of hook-and-eye fastener or a portion of a latch fastener or a portion of a clip fastener.

According to an embodiment of the disposable container, the reservoir volume is up to 20 cm³, alternatively the reservoir volume is up to 15 cm³, further alternatively the reservoir volume is equal to 13 cm³. In any case the reservoir volume of the disposable container is larger than 0 cm³ so that the reservoir volume can contain a drug or a foodstuff.

According to an embodiment of the disposable container, the container comprises a rip-open venting closure sealing a venting opening of the reservoir volume.

According to an embodiment of the disposable container, the disposable container is an envelope or pouch, preferably the envelope or pouch is pre-filled with a foodstuff or a drug as outlined above.

The present invention further relates to a body-wearable medical device, wherein an object of the present invention is solved by a body-wearable medical device comprising a disposable container fastener configured for detachably fastening a disposable container to the body-wearable medical-device. The body-wearable medical device can be configured as described above in the context of the medical device system. By way of example only, the disposable container can be configured as described in the context of the medical device system above.

According to an embodiment of the body-wearable medical device is a body wearable drug infusion pump, in a preferred embodiment a body wearable drug infusion patch pump, including an insulin pump. Alternatively, the body-wearable medical device may be a body wearable in vitro or in vivo analyte sensor including a continuous glucose measurement device. Other body wearable medical devices include a body wearable handheld remote control of a controlled analyte sensor device or drug infusion device, such as a smartphone a wearable such as a watch or a proprietary remote control such of the above such as a diabetes management device.

According to an embodiment of the body-wearable medical device, the disposable container fastener is or comprises a mechanical, non-adhesive disposable container fastener.

According to an embodiment of the body-wearable medical device, the disposable container fastener is or comprises a disposable container bracket configured for at least partially embracing a container.

According to an embodiment of the body-wearable medical device, the disposable container fastener comprises a circumferential edge, which, in a state of a disposable container fastened to the body-wearable medical device, circumferentially contacts the disposable container.

According to an embodiment of the body-wearable medical device, the disposable container fastener is configured to interoperate with body-wearable device fastener of a disposable container to detachably fasten the disposable container to the body-wearable medical device.

According to an embodiment of the body-wearable medical device, the disposable container fastener forms a portion of a pair of hook-and-loop fastener or a portion of a pair of hook-and-eye fastener or a portion of a latch fastener or a portion of a clip fastener.

Insofar as in the foregoing as well as in the following detailed description of embodiments and claims, features of the disposable container described in the context of a body-wearable medical device system are applicable to the disposable container on its own, i.e. irrespective of the presence of a body-wearable medical device. Likewise, insofar as in the foregoing as well as in the following detailed description of embodiments and claims, features of the body-wearable medical device described in the context of a body-wearable medical device system are applicable to the body-wearable medical device on its own, i.e. irrespective of the presence of a disposable container.

Further advantages, features and technical effects of the present invention are apparent from the following description of embodiments and the accompanying figures.
- Figure 1: shows a schematic view of a medical device system according to an embodiment of the present invention; and
- Figure 2: shows a schematic view of a medical device system according to another embodiment of the present invention.

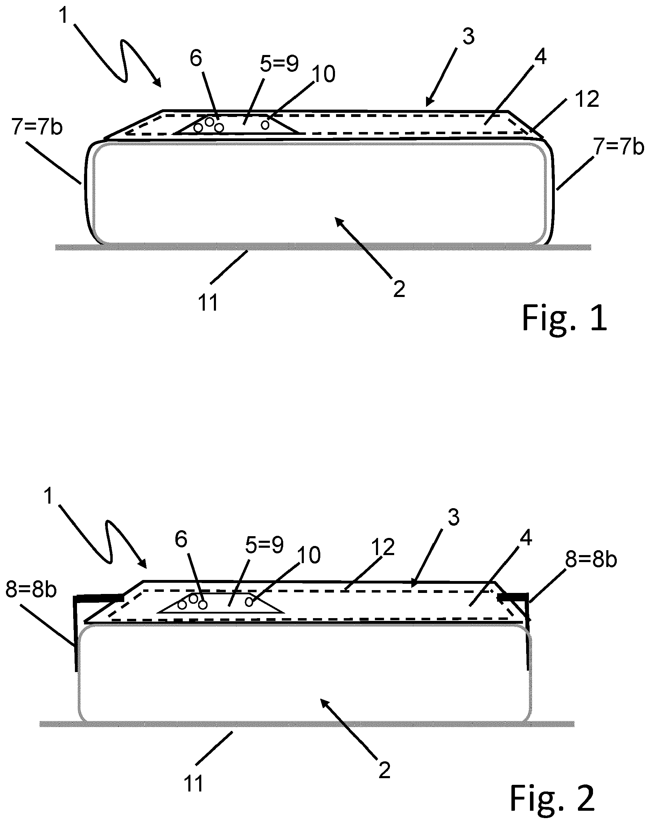

Figure 1 shows a schematic view of a medical device system 1 according to an embodiment of the present invention. The medical device system 1 comprises a body-wearable medical device 2 and disposable container 3. The schematic view shows the disposable container 3 in a state detachably fastened to the body-wearable medical device 2. The body-wearable medical device 2 is visualized in a state attached to a skin 11 of the patient. The body-wearable medical device 2 is a drug infusion pump, such as an insulin pump, or continuous glucose measurement device.

The disposable container 3 comprises a volume reservoir 4 containing a foodstuff or a drug. An opening 6 of the disposable container 3 provides access to the volume reservoir 4. When opened the foodstuff or the drug can be withdrawn from the volume reservoir 4 through the opening 6. For a smooth withdrawal of foodstuff or drug particularly in liquid or gel state, the disposable container 3 further comprises a venting opening 10. The venting opening 10 allows a flow of air flowing into the volume reservoir 4 when the foodstuff or the drug is withdrawn out of the volume reservoir 4 via the opening 6. The venting opening 10 is optional.

Further optional, in the shown embodiment, the opening 6 for withdrawing a foodstuff or a drug out of the volume reservoir 4 is formed by multiple holes.

Initially the opening 6 and the venting opening 10 are sealed by a single, combined tamper-evident closure comprising the rip-open venting closure 9 and the tamper-evident closure 5. Accordingly, the rip-open venting closure 9 sealing the venting opening 10, and the tamper-evident closure 5 form a single element. The tamper-evident closure 5 seals the opening 6 for withdrawing foodstuff or drug out of the volume reservoir 4

The disposable container 3 comprises a body-wearable medical device fastener 7. The body-wearable medical device fastener 7 is configured to detachably fasten the disposable container 3 to the body-wearable medical device fastener 7. In the present embodiment the body-wearable medical device fastener 7 is a mechanical, non-adhesive medical device fastener 7a forming a medical device bracket 7b. In a state in which the disposable container 3 is fastened to the body-wearable medical device 2, the medical device bracket 7b at least partially embraces the body-wearable medical device 2.

Furthermore, the disposable container 3 comprises a circumferential edge 12, which in a state the disposable container 3 is detachably fastened to the body-wearable medical device 2 circumferentially contacts the body-wearable medical device 2.

Figure 2 shows another embodiment of a medical device system 1 according to the present invention. The embodiment according to figure 2 differs from the embodiment shown in figure 1 by having disposable container fastener 8 instead of having body-wearable medical device fastener 7. The disposable container fastener 8 is configured to detachable fasten the disposable container 3 to the body-wearable medical device 2. The disposable container fastener 8 is a mechanical, non-adhesive disposable container fastener 8a in form of a disposable container bracket 8b, which at least partially embrace the disposable container 3, when being detachable mounted to the body-wearable medical device 2.

### List of reference numbers

- 1: medical device system
- 2: body-wearable medical device
- 3: disposable container
- 4: reservoir volume
- 5: tamper-evident closure
- 6: opening
- 7: body-wearable medical device fastener
- 7a: mechanical, non-adhesive medical device fastener
- 7b: medical device bracket
- 8: disposable container fastener
- 8a: mechanical, non-adhesive disposable container fastener
- 8b: disposable container bracket
- 9: rip-open venting closure
- 10: venting opening
- 11: Body skin
- 12: circumferential edge of the disposable container 3

## Claims

1. Medical device system (1) comprising
• a body-wearable medical device (2), and
• a disposable container (3) comprising
∘ a reservoir volume (4) configured for storing a foodstuff or a drug, and
∘ a tamper evident closure (5) which seals an opening (6) in the disposable container (3), wherein the opening (6) provides access to the reservoir volume (4)
• wherein the disposable container (3) comprises a body-wearable medical device fastener (7) configured for detachable fastening the disposable container (3) to the body-wearable medical device (2), and/or
• the body-wearable medical device (2) comprises a disposable container fastener (8) configured for detachable fastening the body-wearable medical device (2) to the disposable container (3).

2. Medical device system (1) according to claim 1, wherein the body-wearable medical device fastener (7) is or comprises a mechanical, non-adhesive medical device fastener (7a) and/or the disposable container fastener (8) is or comprises a mechanical, non-adhesive disposable container fastener (8a).

3. Medical device system (1) according to claim 1 or 2, wherein the body-wearable medical device fastener (7) is or comprises a medical device bracket (7b) configured for at least partially embracing the body-wearable medical device (2) and/or the disposable container fastener (8) is or comprises a disposable container bracket (8b) configured for at least partially embracing the disposable container (3).

4. Medical device system (1) according to any one of claims 1 to 3, wherein the disposable container (3) comprises a circumferential edge (12), which, in a state of the disposable container (3) being fastened to the body-wearable medical device (2) circumferentially contacts the body-wearable medical device (2).

5. Medical device system (1) according to any one of claims 1 to 4, wherein the disposable container fastener (8) and the body-wearable medical device fastener (7) are configured to interoperate to detachably fasten the disposable container (3) to the body-wearable medical device (2).

6. Medical device system (1) according to claim 5, wherein the disposable container fastener (8) and the body-wearable medical device fastener (7) form a pair of hook-and-loop fastener or a pair of hook-and-eye fastener or a latch fastener or clip fastener.

7. Medical device system (1) according to any one of claims 1 to 6, wherein the reservoir volume (4) is up to 20 cm³, alternatively the reservoir volume (4) is up to 15 cm³, further alternatively the reservoir volume (4) is equal to 13 cm³.

8. Medical device system (1) according to any one of claims 1 to 7, wherein the disposable container (3) comprises a rip-open venting closure (9) sealing a venting opening (10) of the reservoir volume (4).

9. Disposable container (3) comprising
• a reservoir volume (4) configured for storing a foodstuff or a drug,
• a tamper evident closure (5) which seals an opening (6) in the disposable container (3), the opening (6) provides access to the reservoir volume (4),
• wherein the disposable container (3) comprises a body-wearable medical device fastener (7) configured for detachable fastening the disposable container (3) to a body-wearable medical device (2).

10. Disposable container (3) according to claim 9, wherein the body-wearable medical device fastener (7) is or comprises a mechanical, non-adhesive medical device fastener (7a).

11. Disposable container (3) according to claim 9 or 10, wherein the body-wearable medical device fastener (7) is or comprises a medical device bracket (7b) configured for at least partially embracing the body-wearable medical device (2).

12. Disposable container (3) according to any one of claims 9 to 11, wherein the disposable container (3) comprises a circumferential edge, which, in a state of the disposable container (3) fastened to the body-wearable medical device (2), circumferentially contacts the body-wearable medical device (2).

13. Body-wearable medical device (2) comprising a disposable container (3) fastener configured for detachable fastening a disposable container (3) to the body-wearable medical-device (2).

14. Body-wearable medical device (2) according to claim 13, **characterized by** being an insulin pump or a continuous glucose measurement device or a handheld diabetes management device.

15. Body-wearable medical device (2) according to claim 13 or 14, wherein the disposable container fastener (8) is or comprises a mechanical, non-adhesive disposable container fastener (8a).
